(19) Europäisches Patentamt
European Patent Office
Office européen des brevets

(11) Publication number: **0 275 591 B1**

(12) **EUROPEAN PATENT SPECIFICATION**

(45) Date of publication of patent specification: **29.01.92**

(51) Int. Cl.5: **C07C 69/593**, C07C 67/38

(21) Application number: **87202536.6**

(22) Date of filing: **15.12.87**

(54) **Process for the selective oxidative carbonylation of conjugated dienes.**

(30) Priority: **24.12.86 NL 8603301**

(43) Date of publication of application:
**27.07.88 Bulletin 88/30**

(45) Publication of the grant of the patent:
**29.01.92 Bulletin 92/05**

(84) Designated Contracting States:
**AT BE DE FR GB IT NL**

(56) References cited:
**EP-A- 0 231 044**
**DE-A- 2 512 062**
**GB-A- 2 064 353**
**US-A- 4 189 599**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Drent, Eit**
**Badhuisweg 3**
**NL-1031 CM Amsterdam(NL)**

EP 0 275 591 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the selective oxidative carbonylation of conjugated dienes, and in particular, to an oxidative carbonylation wherein alkene polycarboxylic esters are prepared by the reaction of a diene, carbon monoxide and a compound comprising a hydroxyl group in the presence of a metal catalyst of the platinum group and a quinone derivative as oxidizing agent. The present invention also relates to catalyst systems employed therewith.

From US patent specification No. 4,575,562 a process is known for the preparation of dimethyl adipate, which comprises the reaction of 1,3-butadiene with carbon monoxide under reactive temperature and pressure conditions in the presence of methanol, a catalyst which comprises a compound of the platinum metal group in a high oxidation state and an organic oxidizing agent such as a quinone, a dehydrating agent and preferably in the presence of one or more ligands, in order to form inter alia dimethyl hex-3-ene dioate, followed by hydrogenation of the dimethyl hex-3-ene dioate under formation of dimethyl adipate. This reaction should preferably be carried out in the presence of palladium compounds. Preferred examples of ligands to be employed are: triphenyl phosphine, tri(p-methoxyphenyl) phosphine, tri(p-fluorophenyl) phosphine, tributyl phosphine, triphenyl arsine, triethyl arsine, benzonitrile, acetonitrile, propionitrile, valeronitrile, succinonitrile, glutaronitrile, triphenyl phosphite, lithium chloride, sodium bromide, lithium iodide, potassium iodide and copper chloride. Furthermore, a small quantity of an acid selected from acetic acid, trifluoroacetic acid, sulphuric acid, hydrochloric acid, hydrobromic acid or hydroiodic acid could also be applied within this process.

From the butadiene conversion and the selectivity for dimethyl hex-3-ene dioate as reported in the examples of this patent specification, however, it will be clear to a person skilled in the art that this process can certainly not yet be considered as a real selective preparation with a high yield (>30%, calculated on butadiene) of these alkene dicarboxylic acid esters desired, which are becoming increasingly important as starting materials for further chemical synthesis such as the preparation of adipates. Moreover, this process is carried out at relatively high pressures (34-350 atm) and temperatures (60-190 °C), which makes additional demands on the apparatus to be used and thus leads to higher costs.

It is also known from International Patent Specification WO 80/00250 to convert conjugated diolefins (for example 1,3-butadiene) into esters of alkene polycarboxylic acids (for example dimethyl hex-3-ene dioate) by reacting them with carbon monoxide and an alcohol (for example benzyl alcohol) in the presence of a palladium catalyst, a copper(II) salt and a base, whereupon this unsaturated diester can be hydrolyzed and hydrogenated or vice versa in order to prepare a corresponding linear di-acid (for example adipic acid). The copper(II) compound should be applied in a quantity which is sufficient to oxidize palladium(O) formed by this process back to palladium(II), and the quantity of nucleophilic base should be at least one molar equivalent of the copper(II) salt. As a copper salt, preferably copper chloride is used. Preferred bases are alkali and alkaline earth metal salts of carboxylic acids or carbonates such as sodium acetate, potassium acetate, sodium propionate, sodium butyrate, sodium carbonate or amines such as triethylamine or lutidine.

From the examples of the process described hereinabove, it will be clear to a person skilled in the art that also this process cannot be considered as a really selective high-yielding process for the preparation of the alkene dicarboxylic acid esters desired, such as dimethyl hex-3-ene dioate. Furthermore, the aforesaid process is characterized by relatively long reaction times (144 hours in example I). It is therefore an object of the present invention to provide a selective conversion of butadiene or homologues thereof to the alkene dicarboxylic acid diester desired, such as diethyl or dimethyl hex-3-ene dioate and/or dimethyl hex-2-ene dioate in order to supply the ever growing need for cheaper fine chemicals as starting compounds for further chemical syntheses.

As a result of extensive and prolonged research and development, a process has now surprisingly been found for the selective preparation of compounds according to the formulae:

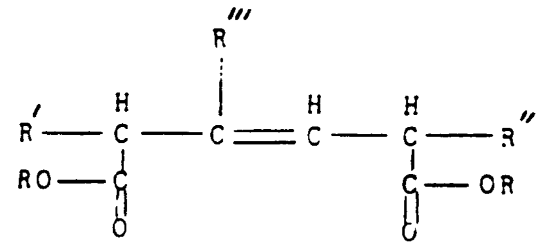

I

and/or

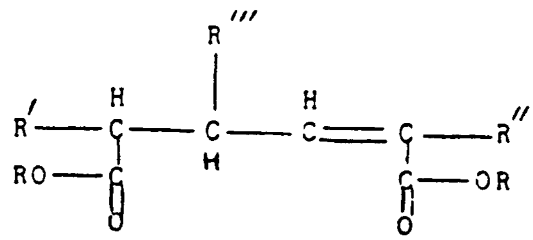

II

where R represents a lower alkyl or aralkyl group, where R′ and R″ each independently represent hydrogen, a lower alkyl, aryl or aralkyl group, and where R‴ represents hydrogen or a lower alkyl group, by means of oxidative carbonylation of a conjugated diene with carbon monoxide and an alcohol in a quantity of at least 2 mol equivalents per mol diene, in the presence of a catalyst system comprising at least the following components:

(a) a compound of a metal, selected from one or more platinum group metals,

(b) an oxidizing agent in the form of quinone and/or derivatives thereof, and

(c) a compound of a metal, selected from manganese or vanadium, as cocatalyst.

It will be appreciated that the expression "a lower alkyl group" refers to groups of 1-4 carbon atoms, the expression "an aryl group" to optionally substituted phenyl or naphthyl groups, and the expression "an aralkyl group" to phenyl or naphthyl groups, substituted with a lower alkyl group.

In preferred embodiments of the process according to the present invention, compounds are formed in which R is a methyl or ethyl group and in which R′, R″ and R‴ are hydrogen or a methyl group. More preferably, a diester of hex-3-ene dicarboxylic acid and/or hex-2-ene dicarboxylic acid is formed by means of oxidative carbonylation of a conjugated diene and in particular 1,3-butadiene.

Besides the aforesaid components of the catalyst system, small quantities of one or more dehydrating agents can, if required, also be inlcluded and especially for the embodiments wherein the reaction is carried out in a reactor to trap water that is formed in situ and adversely affects this reaction.

The compound of a platinum group metal may comprise additional ligands, but it has been found that this is not necessary for optimum conversions.

Oxygen can be used to regenerate the oxidizing agent and can be added to the reactor together with carbon monoxide or in a separate reactor. The reaction can be performed in a batchwise continuous or semi-continuous manner. By "semi-continuous" is meant a reaction system in which the reactants are introduced continuously into a reactor, but no reaction products are withdrawn from the reactor until the reaction is completed. The alcohol should be applied in a quantity which is at least stoichiometric with respect to the quantity of dicarboxylic acid ester to be formed. Preferably, methanol or ethanol is used as alcohol in a quantity of 2 to 10 mol per mol of diester to be formed. Small quantities of dehydrating agents can optionally be added to the reaction mixture. These serve to maintain the desired water-free conditions. Examples of suitable dehydrating agents are methyl orthoformate, metaboric acid, 2,2-dimethoxypropane, 1,4-dimethoxycyclohexane, methyl vinyl ether and 1-ethoxy-cyclohexene.

By "metal compound of the platinum group" is meant compounds of ruthenium, rhodium, palladium, osmium, iridium, platinum and mixtures thereof. Suitable examples of platinum group metal compounds are platinum bromide, platinum chloride, palladium iodide, palladium chloride, palladium bromide and palladium acetate. Metals or metal oxides can be used directly if the reaction conditions are such that a suitable catalytic metal salt is formed during the reaction. Preferably, a palladium compound is used as metal compound in the conversion according to the present invention, and in particular palladium acetate. The palladium group metal catalyst can be acting homogeneously or heterogeneously. The heterogeneous form can be present as a slurry or impregnated in silicon oxide, aluminium oxide, carbon, etc. or natural or synthetic zeolites or other suitable inert materials. The metal catalyst can also be present in a polymer-bound form. The quantity of platinum group metal catalyst component ranges from 0.001 to 10 gram-atom per 100 mol, and preferably between 0.01 and 1.0 gram-atom per 100 mol diene.

The oxidizing agent used to keep the platinum group metal in its oxidized state can for example comprise:
1,4 benzoquinone, 2,5-dichloro-1,4-benzoquinone, 2,6-dichloro-1,4-benzoquinone, tetrachloro-1,4-benzoquinone (chloranil), 2,3-dicyano-1,4-benzoquinone, tetramethyl-1,4-benzoquinone, 2,3-dichloro-5,6-dicyano-1,4-benzoquinone, 2,5-diphenyl-1,4-benzoquinone, 1,4-naphthoquinone, 2,3-dichloro-1,4-naphthoquinone, 2,3-dimethyl-1,4-naphthoquinone or mixtures thereof. The aforesaid compounds can be considered as internal oxidizing agents. The preferred internal oxidizing agent is tetrachloro-1,4-benzoquinone (chloranil). Optionally, quinone/hydroquinone mixtures can also be used. The quinone, optionally mixed with hydroquinone, can be adsorbed on an inert material, such as natural or synthetic zeolites, or can be incorporated in an organic polymer. The molar ratio of the internal oxidizing agent to the platinum group metal component can vary between wide limits and generally lies in the range of 100-700 and preferably between 300 and 600. Examples of suitable manganese or vanadium compounds are manganese chloride, vanadium chloride, manganese bromide, vanadium bromide, manganese acetate, vanadium acetate and mixtures thereof. Preferably, manganese chloride or vanadium chloride are used.

The ratio of the quantities of cocatalyst to platinum group metal catalyst is not critical and can vary between wide limits. Preferably, 5-50 mol of the manganese or vanadium salt per gram-atom palladium is used in order to atain a high selectivity in combination with an attractive conversion rate. High selectivities (>80%) in combination with high conversion rates are obtained if more than 10 mol and less than 45 mol of this cocatalyst per gram-atom palladium is used.

The process according to the present invention does not require the use of an additional solvent if a large excess of one of the reactants, usually the alcohol or one of the reaction products forms a suitable liquid phase. According to the preferred embodiments of the present process, however, an additional solvent is used. Any inert solvent can be used for this purpose. This can, for example, be chosen from sulphones, for example diisopropyl sulphone, tetrahydrothiophene-1,1-dioxide (sulfolan), 2-methyl-4-butyl sulfolan, 3-methyl sulfolan, 2-methyl-4-butyl sulfolan; nitriles such as acetonitrile, propionitrile, benzonitrile; hydrocarbons such as benzene, toluene, xylenes, isooctane, n-hexane, cyclopentane, cyclohexane, cyclooctane; esters such as methyl acetate, ethyl acetate, methyl propionate, butyl propionate, methyl benzoate, dimethyl adipate and butyrolactone; ketones such as acetone, acetophenone, 2-butanone, cyclohexanone or methyl isobutyl ketone; and ethers such as anisole, 2,5,8-trioxanone (also referred to as diglym), diphenyl ether and diisopropyl ether. Preferably, solvents of the ether type, such as diglym, are used.

The process according to the present invention enables relatively mild reaction conditions to be used. Temperatures in the range of 50° to 200°C and in particular in the range of 50° to 150°C are suitable and temperatures in the range of 80° to 125°C are preferred. In the process according to the present invention, oxygen can be used to maintain the internal oxidizing agent concentration at a high enough level to keep the platinum group metal complex in the higher oxidation state desired, i.e. in the catalytically active form for the carbonylation. Oxygen can be added with carbon monoxide, in which case precautions to prevent the formation of explosive oxygen/carbon monoxide mixtures must be taken, or in a separate reactor. According to another embodiment, carbon monoxide and oxygen can be added to the reaction in a suitable alternating manner.

It will be appreciated that no water is formed during the actual carbonylation reaction of the process, but that water is formed during the oxidative regeneration of the quinone derivative from the corresponding hydroquinone derivative. If the carbonylation and the quinone regeneration take place in the same reactor, the addition of sufficient dehydrating agent is desirable in order to minimize the occurrence of side reactions, including the oxidation of butadiene or homologues or derivatives thereof. If the carbonylation and the quinone regeneration are carried out in separate reactors, the quinone-containing feedstock of the carbonylation reactor has to be practically water-free. The dehydration required can be carried out with the aid of one or more of the same series of the aforesaid dehydration agents or by passing the quinone-containing feedstock through a suitable solid dehydrating agent or by removing the water by distillation.

Carbon monoxide can be used in a practically pure form or mixed with oxygen and/or one or more inert gases such as nitrogen or a noble gas. The carbon monoxide pressure applied will in general be lower than that according to hitherto known processes. Pressures of 50 bar and above are preferred.

The molar ratio of the alcohol to the conjugated diene and in particular butadiene can vary between wide limits and generally lies in the range of 2:1 - 10:1. The alcohol can be aliphatic, cycloaliphatic or aromatic and can optionally carry one or more inert substituents. Alkanols such as methanol, ethanol, propanol, 2,2-dihydroxymethyl-1-butanol and benzyl alcohol form suitable starting compounds. Of these, methanol and ethanol are preferred.

It will be clear from the examples given hereinafter that, with the use of manganese or vanadium compounds as cocatalyst, the average conversion rate and selectively found for the conversion of the alkanol to the diester of hex-3-ene dicarboxylic acid and/or hex-2-ene dicarboxylic acid are surprisingly attractive relative to those obtained by the application of copper chloride, or chromium or iron compounds which an expert would have considered to perform similarly.

It will be clear that the present invention also relates to catalyst systems for the oxidative carbonylation of a conjugated diene with carbon monoxide and an alcohol, which comprise at least the following components:

a) a compound of a metal, selected from one or more platinum group metals,
b) an oxidizing agent in the form of quinone and/or derivatives thereof, and
c) a compound of a metal, selected from manganese or vanadium, as cocatalyst.

Besides the aforesaid components of the catalyst system, small quantities of one or more dehydrating agents, as described hereinabove, can be included.

Suitable platinum group metal compounds, oxidizing agents and compounds as cocatalyst are those as described hereinabove.

Preferably, 5-50 mol of the manganese or vanadium compound (salt) per gram-atom platinum group metal catalyst component is incorporated in catalyst systems according to the present invention. The molar ratio of the internal oxidizing agent to the platinum group metal component in said catalyst systems generally lies between 100 and 700, and preferably between 300 and 600. The catalyst systems can also comprise an additional solvent as descrbed hereinabove, preferably diglym.

The invention will now be illustrated by the following Examples.

Example 1

A 250 ml magnetically stirred Hastelloy C [R]autoclave was filled with 40 ml diglym, 10 ml butadiene, 15 ml ethanol, 0.1 mmol palladium acetate, 50 mmol chloranil and 4 mmol manganese chloride ($MnCl_2$). The autoclave was filled with carbon monoxide to a pressure of 60 bar, closed and heated to a temperature of 110 °C. After a reaction time of 2 hours, the contents of the autoclave were analyzed by means of gas-liquid chromatography. The selectivity of the conversion of ethanol to diethyl hex-3-ene dioate and diethyl hex-2-ene dioate was 89%, while an average conversion rate of 200 mol/gat.Pd.hour was found.

Example 2

In a virtually analogous manner to that described in Example 1, an experiment was carried out wth a catalyst system comprising 2 mmol instead of 4 mmol $MnCl_2$. The selectivity of the conversion of ethanol to diethyl hex-3-ene dioate and diethyl hex-2-ene dioate was 85%, while an average conversion rate of 155 mol/gat.Pd.hour was found.

Example 3

In a virtually analogous manner to that described in Example 1, an experiment was carried out in which carbon monoxide pressure was 70 bar instead of 60 bar. The selectivity of the conversion of ethanol to diethyl hex-3-ene dioate and diethyl hex-2-ene dioate was 91%, while an average conversion rate of 170 mol/gat.Pd.hour was found.

Example 4

In a virtually analogous manner to that described in Example 1, an experiment was carried out with a catalyst system comprising 2 mmol vanadium chloride ($VCl_3$) instead of 4 mmol $MnCl_2$ and using a carbon monoxide pressure of 50 instead of 60 bar. The selectivity of the conversion of ethanol to diethyl hex-3-ene dioate and diethyl hex-2-ene dioate was 80%, while an average conversion rate of 130 mol/gat.Pd.hour was found.

In a virtually analogous manner to that described in Example 1, comparative experiments were carried out with catalyst systems comprising 2 mmol copper chloride ($CuCl_2$), 2 mmol chromium acetoacetate (Cr-(Acac)$_3$) and 2 mmol ferric chloride ($FeCl_3$) instead of 4 mmol $MnCl_2$, while the reaction time for the experiment with $CuCl_2$ was 5 hours for the others 2 hours. The selectivity of the conversions was 72%, 90%

[R] (the word Hastelloy is a Registered Trade Mark)

and 30% respectively, while average conversion rates of 100, 50 and 150 mol/gat.Pd.hour respectively were found. Chromium acetoacetate was used because of the very poor solubility of chromium chloride.

An analogous experiment without a cocatalyst gave a selectivity of 85% and an average conversion rate of 75 mol/gat.Pd.hour. These results demonstrate clearly the surprising properties of manganese and vanadium compounds as cocatalyst relative to copper, chromium, and iron compounds.

## Claims

**1.** A process for the selective preparation of compounds according to the formulae:

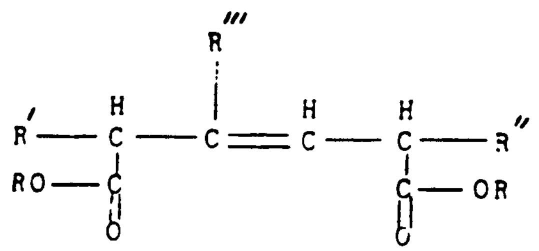

I

and/or

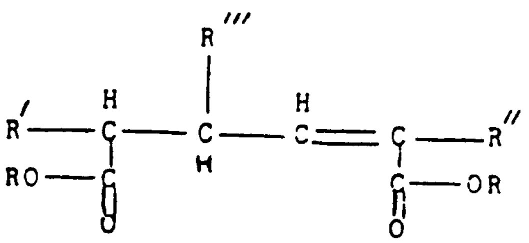

II

where R represents a lower alkyl or aralkyl group, where $R'$ and $R''$ each independently represent hydrogen, a lower alkyl, aryl or aralkyl group, and where $R'''$ represents hydrogen or a lower alkyl group, by means of oxidative carbonylation of a conjugated diene with carbon monoxide and an alcohol in a quantity of at least 2 mol equivalents per mol diene, in the presence of a catalyst system comprising at least the following components:

(a) a compound of a metal, selected from one or more platinum group metals,

(b) an oxidizing agent in the form of quinone and/or derivatives thereof, and

(c) a compound of a metal, selected from manganese or vanadium, as cocatalyst.

**2.** A process according to claim 1, characterized in that compounds are formed in which R is a methyl or ethyl group and in which $R'$, $R'$ and $R'''$ are hydrogen or a methyl group.

**3.** A process according to claim 1 or 2, characterized in that 1,3-butadiene is converted.

**4.** A process according to any one of claims 1-3, characterized in that besides the aforesaid components of the catalyst system, small quantities of one or more dehydrating agents are included.

**5.** A process according to claim 4, characterized in that methyl orthoformate, metaboric acid, 2,2-dimethoxypropane, 1,4-dimethoxycyclohexane, methyl vinyl ether or 1-ethoxy-cyclohexene is used as dehydrating agent.

**6.** A process recording to any one of claims 1-5, characterized in that as platinum group metal compounds, palladium iodide, palladium chloride, palladium bromide, palladium acetate, platinum bromide or platinum chloride are used.

**7.** A process according to any one of claims 1-6, characterized in that the quantity of the platinum group metal catalyst component used ranges from 0.001 to 10 gram-atom per 100 mol diene.

**8.** A process according to claim 7, characterized in that the quantity of the platinum group metal catalyst

component used ranges from 0.01 to 1.0 gram-atom per 100 mol diene.

**9.** A process according to any one claims 1-8, characterized in that the oxidizing agent is used, selected from:
1,4 benzoquinone, 2,5-dichloro-1,4-benzoquinone, 2,6-dichloro-1,4-benzoquinone, tetrachloro-1,4-benzoquinone (chloranil), 2,3-dicyano-1,4-benzoquinone, tetramethyl-1,4-benzoquinone, 2,3-dichloro-5,6-dicyano-1,4-benzoquinone, 2,5-diphenyl-1,4-benzoquinone, 1,4-naphthoquinone, 2,3-dichloro-1,4-naphthoquinone, 2,3-dimethyl-1,4-naphthoquinone or mixtures thereof.

**10.** A process according to any one of claims 1-9, characterized in that the molar ratio of the internal oxidizing agent to the platinum group metal component lies between 100 and 700 and preferably between 300 and 600.

**11.** A process according to any one of claims 1-10, characterized in that as a cocatalyst a compound is used, selected from:
manganese chloride, vanadium chloride, manganese bromide, vanadium bromide, manganese acetate, vanadium acetate or mixtures thereof.

**12.** A process according to claim 11, characterized in that 5-50 mol of the manganese vanadium salt is used per gram-atom platinum group metal catalyst component.

**13.** A process according to any one of clams 1-12, characterized in that the molar ratio of alcohol to conjugated diene lies in the range of 2:1 to 10:1.

**14.** A process according to any one of clams 1-13, characterized in that methanol or ethanol is used.

**15.** A process according to any one of clams 1-14, characterized in that it is carried out in the presence of, besides the aforesaid components of the catalyst system, also diglym as additional solvent.

**16.** A process according to any one of clams 1-15, characterized in that the reaction is carried out at temperatures of 80° to 125°C.

**17.** A process according to any one of clams 1-16, characterized in that the reaction is carried out at a pressure of 50 bar or above.

**18.** Catalyst systems for the oxidative carbonylation of a conjugated diene with carbon monoxide and an alcohol, characterized in that the catalyst system comprises at least the following components:
(a) a compound of a metal, selected from one or more platinum grow metals,
(b) an oxidizing agent in the form of quinone and/or derivatives thereof, and
(c) a compound of a metal, selected from manganese or vanadium, as cocatalyst, and that
small quantities of one or more dehydrating agents are present.

**19.** Catalyst systems according to claim 18, characterized in that palladium iodide, palladium chloride, palladium bromide, palladium acetate, platinum bromide and platinum chloride are present as platinum group metal compounds.

**20.** Catalyst systems according to claim 18 or 19, characterized in that 1,4 benzoquinone, 2,5-dichloro-1,4-benzoquinone, 2,6-dichloro-1,4-benzoquinone, tetrachloro-1,4-benzoquinone (chloranil), 2,3-dicyano-1,4-benzoquinone, tetramethyl-1,4-benzoquinone, 2,3-dichloro-5,6-dicyano-1,4-benzoquinone, 2,3-dichloro-1,4-naphthoquinone, 2,5-diphenyl-1,4-benzoquinone, 1,4-naphthoquinone, 2,3-dimethyl-1,4-naphthoquinone or mixtures thereof are incorporated therein as oxidizing agent.

**21.** Catalyst systems according to any one of claims 18-20, characterized in that manganese chloride, vanadium chloride, manganese bromide, vanadium bromide, manganese acetate, vanadium acetate and/or mixtures thereof are present as a cocatalyst.

**22.** Catalyst systems according to any one of claims 18-21, characterized in that 5-50 mol of the manganese or vanadium salt per gram-atom platinum group metal catalyst component is incorporated

herein.

**23.** Catalyst systems according to any one of claims 18-22, characterized in that the molar ratio of the internal oxidizing agent to the platinum group metal component lies between 100 and 700.

**24.** Catalyst systems according to any one of claims 18-23, characterized in that they also comprise diglym.

**Revendications**

**1.** Procédé pour la préparation sélective de composés selon les formules

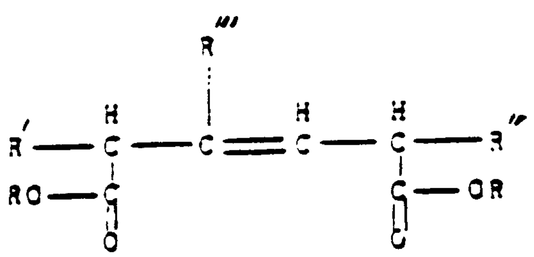

I

et/ou

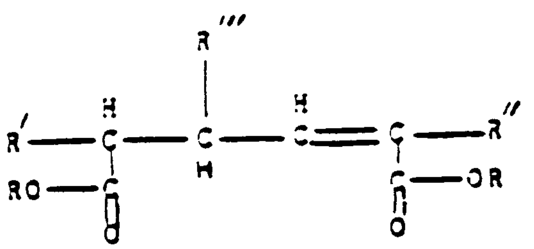

II

où R représente un groupe alkyle inférieur ou aralkyle, où R' et R'' représentent chacun, de façon indépendante, l'hydrogène, un groupe alkyle inférieur, aryle ou aralkyle, et où R''' représente l'hydrogène ou un groupe alkyle inférieur, au moyen de la carbonylation oxydative d'un diène conjugué avec l'oxyde de carbone et un alcool, en quantité d'au moins 2 équivalents molaires par mole de diène, en présence d'un système catalytique comportant au moins les composants suivants :
(a) un composé d'un métal, choisi à partir d'un ou de plusieurs métaux du groupe du platine,
(b) un agent oxydant sous forme de la quinone et/ou de ses dérivés, et
(c) un composé d'un métal, choisi parmi le manganèse ou le vanadium, comme cocatalyseur.

**2.** Procédé selon la revendication 1, caractérisé en ce que l'on forme des composés dans lesquels R est un groupe méthyle ou éthyle et dans lesquels R', R'' et R''' sont l'hydrogène ou un groupe méthyle.

**3.** Procédé selon la revendication 1 ou 2, caractérisé en ce que l'on transforme le 1,3-butadiène.

**4.** Procédé selon l'une quelconque des revendications 1 à 3, caractérisé en ce que, en plus des composés précités du système catalytique, on inclut de petites quantités d'un ou de plusieurs agents deshydratants.

**5.** Procédé selon la revendication 4, caractérisé en ce que l'on utilise comme agent deshydratant l'orthoformiate de méthyle, l'acide métaborique, le 2,2-diméthoxypropane, le 1,4-diméthoxycyclohexane, l'éther méthyl vinylique ou le 1-éthoxy-cyclohexène.

**6.** Procédé selon l'une quelconque des revendications 1 à 5, caractérisé en ce que l'on utilise comme

composés de métaux du groupe du platine l'iodure de palladium, le chlorure de palladium, le bromure de palladium, l' acétate de palladium, le bromure de platine ou le chlorure de platine.

**7.** Procédé selon l'une quelconque des revendications 1 à 6, caractérisé en ce que la quantité du composé catalytique d'un métal du groupe du platine va de 0,001 à 10 atomes-grammes par 100 moles de diène.

**8.** Procédé selon la revendication 7, caractérisé en ce que la quantité de composant catalytique d'un métal du groupe du platine utilisé va de 0,01 à 1,0 atome-gramme par 100 moles de diène.

**9.** Procédé selon l'une quelconque des revendications 1 à 8, caractérisé en ce que l'on utilise un agent oxydant choisi parmi :
la 1,4-benzoquinone, la 2,5-dichloro-1,4-benzoquinone, la 2,6-dichloro-1,4-benzoquinone, la tétrachloro-1,4-benzoquinone (chloranil), la 2,3-dicyano-1,4-benzoquinone, la tétraméthyl-1,4-benzoquinone, la 2,3-dichloro-5,6-dicyano-1,4-benzoquinone, la 2,5-diphényl-1,4-benzoquinone, la 1,4-naphtoquinone, la 2,3-dichloro-1,4-naphtoquinone, la 2,3-diméthyl-1,4-naphtoquinone ou leurs mélanges.

**10.** Procédé selon l'une quelconque des revendications 1 à 9, caractérisé en ce que le rapport molaire de l'agent interne d'oxydation et du composant de métal du groupe du platine est situé entre 100 et 700 et de préférence entre 300 et 600.

**11.** Procédé selon l'une quelconque des revendications 1 à 10, caractérisé en ce que l'on utilise un composé comme cocatalyseur, choisi parmi :
le chlorure de manganèse, le chlorure de vanadium, le bromure de manganèse, le bromure de vanadium, l'acétate de manganèse, l'acétate de vanadium ou leurs mélanges.

**12.** Procédé selon la revendication 11, caractérisé en ce que de 5 à 50 moles du sel de manganèse ou de vanadium sont utilisées par atome-gramme du composant catalytique de métal du groupe du platine.

**13.** Procédé selon l'une quelconque des revendications 1 à 12, caractérisé en ce que le rapport molaire de l'alcool au diène conjugué est dans les limites de 2:1 à 10:1.

**14.** Procédé selon l'une quelconque des revendications 1 à 13, caractérisé en ce que l'on utilise le méthanol ou l'éthanol.

**15.** Procédé selon l'une quelconque des revendications 1 à 14, caractérisé en ce qu'il est mis en oeuvre aussi en présence de diglyme (diglym) comme solvant additionnel, en plus des composants précités du système catalytique.

**16.** Procédé selon l'une quelconque des revendications 1 à 15, caractérisé en ce que la réaction est réalisée à des températures de 80° à 125° C.

**17.** Procédé selon l'une quelconque des revendications 1 à 16, caractérisé en ce que la réaction est effectuée à une pression de 50 bars ou au-dessus.

**18.** Systèmes catalytiques pour la carbonylation oxydative d'un diène conjugué avec l'oxyde de carbone et un alcool, caractérisés en ce que le système catalytique comporte au moins les composants suivants :
(a) un composé d'un métal, choisi parmi un ou plusieurs métaux du groupe du platine,
(b) un agent oxydant sous forme de la quinone et/ou de ses dérivés, et
(c) un composé d'un métal choisi parmi le manganèse ou le vanadium, comme cocatalyseur, et
en ce que de petites quantités d'un ou plusieurs agents deshydratants sont présentes.

**19.** Systèmes catalytiques selon la revendication 18, caractérisés en ce que l'iodure de palladium, le chlorure de palladium, le bromure de palladium, l'acétate de palladium, le bromure de platine et le chlorure de platine sont présents comme composés de métaux du groupe du platine.

**20.** Systèmes catalytiques selon la revendication 18 ou 19, caractérisés en ce que la 1,4-benzoquinone, la 2,5-dichloro-1,4-benzoquinone, la 2,6-dichloro-1,4-benzoquinone, la tétrachloro-1,4-benzoquinone

(chloranil), la 2,3-dicyano-1,4-benzoquinone, la tétraméthyl-1,4-benzoquinone, la 2,3-dichloro-5,6-dicyano-1,4-benzoquinone, la 2,3-dichloro-1,4-naphtoquinone, la 2,5-diphényl-1,4-benzoquinone, la 1,4-naphtoquinone, la 2,3-diméthyl-1,4-naphtoquinone ou leurs mélanges sont incorporés dans ces systèmes comme agent oxydant.

**21.** Systèmes catalytiques selon l'une quelconque des revendications 18 à 20, caractérisés en ce que le chlorure de manganèse, le chlorure de vanadium, le bromure de manganèse, le bromure de vanadium, l'acétate de manganèse l'acétate de vanadium et/ou leurs mélanges sont présents comme cocatalyseur.

**22.** Systèmes catalytiques selon l'une quelconque des revendications 18 à 21, caractérisés en ce que l'on y incorpore 5 à 50 moles du sel de manganèse ou de vanadium par atome-gramme de composant de catalyseur de métal du groupe du platine.

**23.** Systèmes catalytiques selon l'une quelconque des revendications 18 à 22, caractérisés en ce que le rapport molaire de l'agent interne d'oxydation au composant du métal du groupe du platine se trouve entre 100 et 700.

**24.** Systèmes catalytiques selon l'une quelconque des revendications 18 à 23, caractérisés en ce qu'ils comportent aussi du diglyme (diglym).

**Patentansprüche**

**1.** Verfahren zur selektiven Herstellung von Verbindungen entsprechend den Formeln:

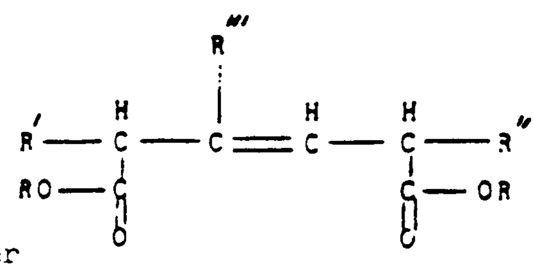

I

und/oder

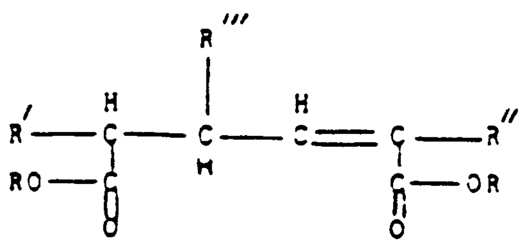

II,

worin

| | |
|---|---|
| R | eine Niederalkyl- oder Aralkylgruppe bedeutet, |
| R' und R" | jeweils unabhängig voneinander Wasserstoff, eine Niederalkyl-, Aryl- oder Aralkylgruppe darstellen und |
| R"' | Wasserstoff oder eine Niederalkylgruppe bedeutet, |

durch oxidative Carbonylierung eines konjugierten Diens mit Kohlenmonoxid und einem Alkohol in einer Menge von wenigstens 2 Moläquivalenten je Mol Dien, in Gegenwart eines Katalysatorsystems, das wenigstens die folgenden Komponenten umfaßt:

(a) eine Verbindung eines Metalles, ausgewählt unter einem oder mehreren Platingruppenmetallen,
(b) ein Oxidationsmittel in Form von Chinon und/oder Derivaten hievon, und
(c) eine Verbindung eines unter Mangan oder Vanadium ausgewählten Metalles als Cokatalysator.

**2.** Verfahren nach Anspruch 1, dadurch gekennzeichnet, daß Verbindungen gebildet werden, worin R eine Methyl- oder Ethylgruppe bedeutet und worin R', R" und R"' Wasserstoff oder eine Methylgruppe

darstellen.

3. Verfahren nach Anspruch 1 oder 2, dadurch gekennzeichnet, daß 1,3-Butadien umgewandelt wird.

4. Verfahren nach einem der Anspüche 1 bis 3, dadurch gekennzeichnet, daß neben den vorstehend genannten Komponenten des Katalysatorsystems geringe Mengen eines oder mehrerer dehydratisierender Mittel eingeschlossen sind.

5. Verfahren nach Anspruch 4, dadurch gekennzeichnet, daß als dehydratisierendes Mittel Methylorthoformiat, Metaborsäure, 2,2-Dimethoxypropan, 1,4-Dimethoxycyclohexan, Methylvinylether oder 1-Ethoxycyclohexen eingesetzt wird.

6. Verfahren nach einem der Ansprüche 1 bis 5, dadurch gekennzeichnet, daß als Platingruppenmetallverbindungen Palladiumiodid, Palladiumchlorid, Palladiumbromid, Palladiumacetat, Platinbromid oder Platinchlorid verwendet werden.

7. Verfahren nach einem der Ansprüche 1 bvis 6, dadurch gekennzeichnet, daß die Menge der verwendeten Platingruppenmetallkatalysatorkomponente von 0,001 bis 10 Gramm-Atom je 100 Mol Dien beträgt.

8. Verfahren nach Anspruch 7, dadurch gekennzeichnet, daß die Menge der verwendeten Platingruppenmetallkatalysatorkomponente von 0,01 bis 1,0 Gramm-Atom je 100 Mol Dien beträgt.

9. Verfahren nach einem der Ansprüche 1 bis 8, dadurch gekennzeichnet, daß ein Oxidationsmittel verwendet wird, das unter 1,4-Benzochinon, 2,5-Dichlor-1,4-benzochinon, 2,6-Dichlor-1,4-benzochinon, Tetrachlor-1,4-benzochinon (Chloranil), 2,3-Dicyano-1,4-benzochinon, Tetramethyl-1,4-benzochinon, 2,3-Dichlor-5,6-dicyano-1,4-benzochinon, 2,5-Diphenyl-1,4-benzochinon, 1,4-Naphthochinon, 2,3-Dichlor-1,4-naphthochinon, 2,3-Dimethyl-1,4-naphthochinon oder Gemischen hievon ausgewählt wird.

10. Verfahren nach einem der Ansprüche 1 bis 9, dadurch gekennzeichnet, daß das Molverhältnis von innerem Oxidationsmittel zu der Platingruppenmetallkomponente zwischen 100 und 700 und vorzugsweise zwischen 300 und 600 beträgt.

11. Verfahren nach einem der Ansprüche 1 bis 10, dadurch gekennzeichnet, daß als Cokatalysator eine Verbindung, ausgewählt unter Manganchlorid, Vanadiumchlorid, Manganbromid, Vanadiubromid, Manganacetat, Vanadiumacetat oder Gemischen hievon verwendet wird.

12. Verfahren nach Anspruch 11, dadurch gekennzeichnet, daß 5 bis 50 Mol des Mangan- oder Vanadiumsalzes je Gramm-Atom Platingruppenmetallkatalysatorkomponente verwendet werden.

13. Verfahren nach einem der Ansprüche 1 bis 12, dadurch gekennzeichnet, daß das Mol-Verhältnis von Alkohol zu konjugiertem Dien im Bereich 2:1 bis 10:1 beträgt.

14. Verfahren nach einem der Ansprüche 1 bis 13, dadurch gekennzeichnet, daß Methanol oder Ethanol verwendet wird.

15. Verfahren nach einem der Ansprüche 1 bis 14, dadurch gekennzeichnet, daß es, zusätzlich zur Gegenwart der vorstehend genannten Komponenten des Katalysatorsytems, in Anwesenheit von Diglyme als zusätzlichem Lösungsmittel ausgeführt wird.

16. Verfahren nach einem der Ansprüche 1 bis 15, dadurch gekennzeichnet, daß die Umsetzung bei Temperaturen von 80 bis 125 °C ausgeführt wird.

17. Verfahren nach einem der Ansprüche 1 bis 16, dadurch gekennzeichnet, daß die Umsetzung bei einem Druck von 50 bar oder darüber ausgeführt wird.

18. Katalysatorsysteme für die oxidative Carbonylierung eines konjugierten Diens mit Kohlenmonoxid und einem Alkohol, dadurch gekennzeichnet, daß das Katelysatorsystem wenigstens die folgenden Komponenten umfaßt:

(a) eine Verbindung eines Metalles, ausgewählt unter einem oder mehreren Platingruppenmetallen,
(b) ein Oxidationsmittel in Form von Chinon und/oder Derivaten hievon, und
(c) eine Verbindung eines unter Mangan oder Vanadium ausgewählten Metalles als Cokatalysator, und daß

kleine Mengen eines oder mehrerer dehydratisierender Mittel zugegen sind.

**19.** Katalysatorsysteme nach Anspruch 18, dadurch gekennzeichnet, daß als Platingruppenmetallverbindungen Palladiumiodid, Palladiumchlorid, Palladiumbromid, Palladiumacetat, Platinbromid und Platinchlorid zugegen sind.

**20.** Katalysatorsysteme nach Anspruch 18 oder 19, dadurch gekennzeichnet, daß als Oxidationsmittel 1,4-Benzochinon, 2,5-Dichlor-1,4-benzochinon, 2,6-Dichlor-1,4-benzochinon, Tetrachlor-1,4-benzochinon (Chloranil), 2,3-Dicyano-1,4-benzochinon, Tetramethyl-1,4-benzochinon, 2,3-Dichlor-5,6-dicyano-1,4-benzochinon, 2,3-Dichlor-1,4-naphthochinon, 2,5-Diphenyl-1,4-benzochinon, 1,4-Naphthochinon, 2,3-Dimethyl-1,4-naphthochinon oder Gemische hievon enthalten sind.

**21.** Katalysatorsysteme nach einem der Ansprüche 18 bis 20, dadurch gekennzeichnet, daß Manganchlorid, Vanadiumchlorid, Manganbromid, Vanadiumbromid, Manganacetat, Vanadiumacetat und/oder Gemische hievon als Cokatalysator zugegen sind.

**22.** Katelysatorsysteme nach einem der Ansprüche 18 bis 21, dadurch gekennzeichnet, daß 5 bis 50 Mol des Mangan- oder Vanadiumsalzes je Gramm-Atom Platingruppenmetallkatalysatorkomponente enthalten sind.

**23.** Katelysatorsysteme nach einem der Ansprüche 18 bis 22, dadurch gekennzeichnet, daß das Mol-Verhältnis von innererm Oxidationsmittel zu der Platingruppenmetallkomponente zwischen 100 und 700 beträgt.

**24.** Katalysatorsysteme nach einem der Ansprüche 18 bis 23, dadurch gekennzeichnet, daß sie auch Diglyme unfassen.